# EUROPÄISCHE PATENTSCHRIFT

(11) **EP 2 673 311 B1**
(45) Veröffentlichungstag und Bekanntmachung des Hinweises auf die Patenterteilung: **10.09.2014**
(21) Anmeldenummer: 12702273.9
(22) Anmeldetag: 06.02.2012
(51) Int. Cl.: C08G 18/10, C08G 18/38, A61L 31/06

(54) **GEWEBEKLEBER AUF BASIS TRIFUNKTIONELLER ASPARTATE**
TISSUE ADHESIVE BASED ON TRIFUNCTIONAL ASPARTATES
ADHÉSIF POUR TISSU À BASE D'ASPARTATE TRIFONCTIONNEL

(30) Priorität: 09.02.2011 EP 11153807
(43) Veröffentlichungstag der Anmeldung: 18.12.2013
(73) Patentinhaber: Bayer Materialscience AG, 51368 Leverkusen (DE)
(72) Erfinder: HECKROTH, Heike, 51519 Odenthal (DE); EGGERT, Christoph, 50733 Köln (DE)
(74) Vertreter: BIP Patents
(86) Internationale Anmeldenummer: PCT/EP2012/051934
(87) Internationale Veröffentlichungsnummer: WO 2012/107386

(56) Entgegenhaltungen:
- EP-A1- 2 145 634
- WO-A2-2008/076707

## Beschreibung

Die vorliegende Erfindung betrifft eine aminofunktionelle Verbindung zur Verwendung in einem Polyharnstoff-System, das insbesondere zum Verschließen, Verbinden, Verkleben oder Abdecken von Zellgewebe vorgesehen ist. Weitere Gegenstände der Erfindung sind ein die erfindungsgemäßen Verbinddung umfassendes Polyharnstoff-System sowie ein Dosiersystem für das erfindungsgemäße Polyharnstoff-System.

Diverse Materialien, die als Gewebekleber eingesetzt werden, sind im Handel erhältlich. Hierzu gehören die Cyanacrylate Dermabond^{®} (Octyl-2-Cyanoacrylat) und Histoacryl Blue^{®} (Butyl-Cyanoacrylat). Voraussetzung für eine effiziente Klebung der Cyanacrylate sind allerdings trockene Untergründe. Bei starken Blutungen versagen derartige Klebstoffe.

Als Alternative zu den Cyanacrylaten stehen biologische Klebstoffe wie zum Beispiel BioGlue^{®}, eine Mischung aus Glutaraldehyd und Bovinem Serumalbumin, diverse kollagen- und gelatinebasierte Systeme (FloSeal^{®}) sowie die Fibrinkleber (Tissucol) zur Verfügung. Diese Systeme dienen in erster Linie der Blutstillung (Hämostase). Neben den hohen Kosten zeichnen sich Fibrinkleber durch eine relative schwache Klebestärke und einen schnellen Abbau aus, so dass sie nur bei kleineren Verletzungen auf nicht gespanntem Gewebe verwendet werden können. Kollagen- und Gelatinebasierte Systeme wie FloSeal^{®} dienen ausschließlich der Hämostase. Zudem besteht, da Fibrin und Thrombin aus humanem-, Collagen und Gelatine aus tierischem Material gewonnen werden, bei biologischen Systemen immer die Gefahr einer Infektion. Biologische Materialien müssen außerdem gekühlt gelagert werden, so dass ein Einsatz in der Notfallversorgung wie z. B. in Katastrophengebieten, bei militärischen Einsetzen etc. nicht möglich ist. Hier steht zur Behandlung traumatischer Wunden QuikClot^{®} oder QuikClot ACS+™ zur Verfügung, welches ein mineralisches Granulat ist, das im Notfall in die Wunde gebracht wird und dort durch Wasserentzug zur Koagulation führt. Im Falle von QuikClot® ist dies eine stark exotherme Reaktion, die zu Verbrennungen führt. QuikClot ACS+™ ist eine Gaze, in die das Salz eingebettet ist. Das System muss zur Blutstillung fest auf die Wunde gedrückt werden.

Aus der WO 2009/106245 A2 ist die Herstellung und Verwendung von Polyharnstoff-Systemen als Gewebekleber bekannt. Die hier offenbarten Systeme umfassen wenigstens zwei Komponenten. Dabei handelt es sich um einen aminofunktionellen Asparaginsäureester und ein isocyanatfunktionelles Prepolymer, das durch Umsetzung von aliphatischen Polyisocyanaten mit Polyesterpolyolen erhältlich ist. Die beschriebenen 2-Komponenten Polyharnstoff-Systeme können als Gewebekleber für den Verschluss von Wunden in menschlichen und tierischen Zellverbänden eingesetzt werden. Dabei kann ein sehr gutes Klebeergebnis erzielt werden.

WO 2008 076707 beschreibt Beschichtungszusammensetzungen die Isocyanatprepolymere und Asparate auf Basis Reaktionsprodukte von Dialkylentriamin und Diethylmaleat enthalten.

Um eine gute Mischbarkeit der beiden Komponenten des Polyharnstoff-Systems sicher zu stellen, sollte die Viskosität der Komponenten bei 23 °C möglichst kleiner als 10.000 mPas sein. Eine entsprechend niedrige Viskosität weisen Prepolymere mit NCO-Funktionalitäten von weniger als 3 auf. Wenn derartige Prepolymere eingesetzt werden, ist es notwendig, als zweite Komponente einen Asparaginsäureester mit einer Aminofunktionalität von mehr als 2 einzusetzen, da ansonsten kein polymeres Netzwerk hergestellt werden kann. Dies ist jedoch erforderlich, damit das Polyharnstoff-System bzw. eine daraus bestehende Klebnaht die gewünschten mechanischen Eigenschaften wie Elastizität und Festigkeit aufweist. Darüber hinaus ist bei der Verwendung difunktioneller Asparaginsäureester nachteilig, dass die Aushärtungszeit bis zu 24 h beträgt, wobei das Polyharnstoff-System selbst nach dieser Zeit in vielen Fällen klebrig bleibt, also nicht tack free ist.

Aufgabe der Erfindung war es daher, eine Isocyanat-reaktive Komponente für ein Polyharnstoff-System bereit zu stellen, die gut mit einem Prepolymer mit einer NCO-Funktionalität von weniger als 3 mischbar ist, eine Aminofunktionalität von mehr als 2 aufweist und mit dem Prepolymer, schnell unter Ausbildung eines dreidimensionalen Polyharnstoff-Netzwerks umgesetzt werden kann. Dabei war als zusätzliche Bedingung zu beachten, dass das ausgehärtete System nach ISO 10993 bei der Anwendung im Menschen oder im Tier keine Zytotoxizität aufweist.

Dieses Aufgabe ist erfindungsgemäß durch eine Verbindung der Formel (I) 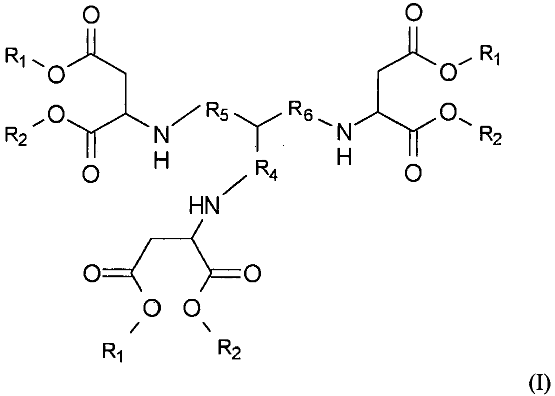 gelöst, in der
- R₁, R₂: jeweils unabhängig voneinander gleiche oder verschiedene organische Reste, die keinen Zerewitinoff-aktiven Wasserstoff aufweisen und
- R₄, R₅, R₆: jeweils unabhängig voneinander gesättigte, linearer oder verzweigter, gegebenenfalls auch in der Kette mit Heteroatomen substituierte, organische Reste, die keinen Zerewitinoff-aktiven Wasserstoff aufweisen
sind.

Die erfindungsgemäße Verbindung lässt sich leicht mit einem Prepolymeren vermischen, da sie bei 23 °C eine Viskosität von weniger als 10.000 mPas aufweist. Darüber hinaus hat sie eine Aminofunktionalität von 3 und ist folglich befähigt mit Prepolymeren einer NCO-Funktionalität von 2 schnell ein dreidimensionales Polyharnstoff-Netzwerk zu bilden. Dieses Netzwerk zeichnet sich durch hohe Elastizität, Festigkeit, Klebstärke und einen Fehlen von Zytotoxizität aus. Außerdem ist das Netzwerk bereits nach kurzer Zeit nicht mehr klebrig, d.h. tack free.

In der Formel (I) können die Reste R₄, R₅, R₆ jeweils unabhängig voneinander lineare oder verzweigte, insbesondere gesättigte, aliphatische C1 bis C 12, bevorzugt C2 bis C10, besonders bevorzugt C3 bis C8 und ganz besonders bevorzugt C3 bis C6 Kohlenwasserstoffreste sein. Derartige aminofunktionelle Verbindungen zeichnen sich dadurch aus, dass sie besonders schnell mit Prepolymeren zu einem stark klebenden, elastischen und festen Polyharnstoff-Netzwerk aushärten.

Gemäß einer weiteren bevorzugten Ausführungsform der erfindungsgemäßen Verbindung sind die Reste R1, R2 jeweils unabhängig voneinander lineare oder verzweigte organische C1 bis C 10, bevorzugt C1 bis C8, besonders bevorzugt C2 bis C6, ganz besonders bevorzugt C2 bis C4 Reste und insbesondere aliphatische Kohlenwasserstoffreste. Auch diese Verbindung zeichnet sich durch eine schnelle Netzwerkbildung bei einer Umsetzung mit einem Prepolymer aus.

Bevorzugt ist ebenfalls, wenn jeweils die Reste R₁ und R₂ gleich und / oder jeweils die Reste R₄, R₅, R₆ gleich sind.

Ganz besonders bevorzugt ist eine Verbindung der Formel (I), bei der R₁ und R₂ jeweils gleichzeitig Methy oder Ethyl und R₄, R₅, R₆ jeweils gleichzeitig Ethyl, Propyl oder Butyl sind.

Ein weiterer Gegenstand der Erfindung ist ein Polyharnstoff-System, umfassend
als Komponente A) isocyanatfunktionelle Prepolymere erhältlich durch Umsetzung von aliphatischen Polyisocyanaten A1) mit
Polyolen A2), die insbesondere ein zahlenmittleres Molekulargewicht von ≥ 400 g/mol und eine mittlere OH-Funktionalität von 2 bis 6 aufweisen können, als Komponente B) eine erfindungsgemäße aminofunktionelle Verbindung gemäß Formel (I)
gegebenenfalls als Komponente C) organische Füllstoffe, die insbesondere eine nach DIN 53019 gemessenen Viskosität bei 23°C im Bereich von 10 bis 6000 mPas aufweisen können,
gegebenenfalls als Komponente D) Umsetzungsprodukte von isocyanatfunktionellen Prepolymeren gemäß Komponente A) mit aminofunktionellen Verbindungen gemäß Komponente B) und/oder organischen Füllstoffen gemäß Komponente C) und
gegebenenfalls als Komponente E) Wasser und/oder ein tertiäres Amin.

Die erfindungsgemäßen Polyharnstoff-Systeme werden durch Mischung der Prepolymere A) mit den aminofunktionellen Verbindung B) sowie gegebenenfalls den Komponenten C), D) und/oder E) erhalten. Das Verhältnis von freien oder blockierten Aminogruppen zu freien NCO-Gruppen beträgt dabei bevorzugt 1:1,5, besonders bevorzugt 1:1. Wasser und/oder Amin werden dabei der Komponente B) bzw. C beigemischt.

Die isocyanatfunktionellen Prepolymere A) sind durch Umsetzung von Polyisocyanaten A1) mit Polyolen A2) gegebenenfalls unter Zusatz von Katalysatoren sowie Hilfs- und Zusatzstoffen erhältlich.

Als Polyisocyanate A1) können beispielsweise monomere aliphatische oder cycloaliphatische Di-oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexa-methylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoat (Lysindiisocyanat) mit C1-C8-Alkylgruppen eingesetzt werden.

Neben den vorstehend genannten monomeren Polyisocyanaten A1) können auch deren höhermolekulare Folgeprodukte mit Uretdion-, Isocyanurat-, Urethan-, Allophanat-, Biuret-, Iminooxadiazindion-oder Oxadiazintrionstruktur sowie deren Mischungen eingesetzt werden.

Bevorzugt werden Polyisocyanate A1) der vorstehend genannten Art mit ausschließlich aliphatisch oder cycloaliphatisch gebundenen Isocyanatgruppen oder deren Mischungen eingesetzt.

Ebenfalls bevorzugt ist, wenn Polyisocyanate A1) der vorstehenden Art mit einer mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 verwendet werden.

Ganz besonders bevorzugt wird Hexamethylendiisocyanat als Polyisocyanat A1) eingesetzt.

Gemäß einer bevorzugten Ausführungsform des erfindungsgemäßen Polyharnstoff-Systems ist vorgesehen, dass die Polyole A2) Polyesterpolyole und/oder Polyester-Polyether-Polyole und/oder Polyetherpolyole sind. Insbesondere bevorzugt sind dabei Polyester-Polyether-Polyole und/oder Polyetherpolyole mit einem Ethylenoxidanteil zwischen 60 und 90 Gew.-%.

Bevorzugt ist auch, wenn die Polyole A2) ein zahlenmittleres Molekulargewicht von 4000 bis 8500 g/mol aufweisen.

Geeignete Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt; wobei als Polyol ethergruppenhaltige Polyole gegebenenfalls in Mischungen mit anderen ethergruppenfreien Polyolen eingesetzt werden.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6, 3-Methyl-pentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligo-tetrahydrofurane mit Hydroxylendgruppen. Selbstverständlich können innerhalb dieser Gruppe auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und/oder Propylenoxid, sowie durch säurekatalysierte, ringöffnende Polymerisation von Tetrahydrofuran, erhalten werden können. Zur ringöffnenden Polymerisation von Epoxiden können entweder Alkalihydroxide oder Doppelmetallcyanidkatalysatoren verwendet werden.

Als Starter für ringöffnende Epoxidpolymerisationen können alle mindestens bifunktionellen Moleküle aus der Gruppe der Amine und der o.g. niedermolekularen Polyole verwendet werden. Typische Vertreter sind 1,1,1-Trimethylolpropan, Glycerin, o-TDA, Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen können.

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Anhydride, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil am gesamten, das Polyetheresterpolyol aufbauenden Reaktionsmischung 10 Gew.% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole ausgeglichen wird.

Die Herstellung der Polyetheresterpolyol erfolgt entsprechend dem Stand der Technik bei erhöhter Temperatur im Bereich von 120 bis 250 °C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt 0,1 bis 3 mg KOH/g und besonders bevorzugt 0,15 bis 2,5 mg KOH/g absinkt.

Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Selbstverständlich können auch Abmischungen von Polyetherpolyolen mit Polyesterpolyolen in beliebigen Verhältnissen eingesetzt werden.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Ebenfalls können Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Zur Herstellung des Prepolymers A) kann das Polyisocyanat A1) mit dem Polyol A2) bei einem NCO/OH-Verhältnis von bevorzugt 4:1 bis 12:1, besonders bevorzugt 8:1 umgesetzt und anschließend der Anteil an nicht umgesetzten Polyisocyanates mittels geeigneter Methoden abgetrennt werden. Üblicherweise wird hierfür die Dünnschichtdestillation verwendet, wobei Prepolymere mit Restmonomergehalten von weniger als 1 Gew.-%, bevorzugt weniger als 0,1 Gew.-%, ganz besonders bevorzugt weniger als 0,03 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Herstellung der Prepolymere A) beträgt dabei bevorzugt 20 bis 120 °C und weiter bevorzugt 60 bis 100 °C.

Die hergestellten Prepolymere haben einen nach DIN EN ISO 11909 gemessenen mittleren NCO-Gehalt von 2 bis 10 Gew.-%, bevorzugt 2,5 bis 8 Gew.-%.

Gemäße einer weiteren Ausführungsform des erfindungsgemäßen Polyharnstoff-Systems können die Prepolymere A) eine mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 aufweisen.

Bei den organischen Füllstoffen der Komponente C) kann es sich bevorzugt um hydroxyfunktionelle Verbindungen, insbesondere um Polyetherpolyole mit sich wiederholenden Ethylenoxid Einheiten handelt.

Vorteilhaft ist auch, wenn die Füllstoffe der Komponente C) eine mittlere OH-Funktionalität von 1,5 bis 3, bevorzugt von 1,8 bis 2,2 und besonders bevorzugt von 2 aufweisen.

Beispielsweise können als organische Füllstoffe bei 23 °C flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Die Viskosität der organischen Füllstoffe gemessen nach DIN 53019 bei 23 °C beträgt bevorzugt 50 bis 4000 mPas, besonders bevorzugt 50 bis 2000 mPas.

In einer bevorzugten Ausführungsform des erfindungsgemäßen Polyharnstoff-Systems werden als organische Füllstoffe Polyethylenglykole eingesetzt. Diese haben bevorzugt ein zahlenmittleres Molekulargewicht von 100 bis 1000 g/mol, besonders bevorzugt 200 bis 400 g/mol.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich Umsetzungsprodukte der Prepolymere A) mit der aminofunktionellen Verbindung B) und/oder den organischen Füllstoffen C), sofern diese amino- oder hydroxyfunktionell sind, in einer separaten Vorreaktion herzustellen und dann als höhermolekulare Härterkomponente einzusetzen.

Bevorzugt werden bei der Vorverlängerung Verhältnisse von isocyanatreaktiven Gruppen zu Isocyanatgruppen von 50 zu 1 bis 1,5 zu 1, besonders bevorzugt 15 zu 1 bis 4 zu 1 eingestellt.

Vorteil dieser Modifizierung durch Vorverlängerung ist, dass das Equivalentgewicht und das Equivalentvolumen der Härterkomponente in größeren Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden kann.

Gemäß einer weiteren bevorzugten Ausführungsform des erfindungsgemäßen Polyharnstoff-Systems ist vorgesehen, dass die Komponente E) ein tertiäre Amine der allgemeinen Formel (II) 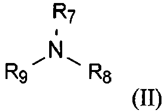 enthält, in der
- R₇, R₈, R₉: unabhängig voneinander Alkyl- oder Heteroalkyreste mit Heteroatomen in der Alkylkette oder an deren Ende sein können, oder R₇ und R₈ gemeinsam mit dem sie tragenden Stickstoffatom einen aliphatischen, ungesättigten oder aromatischen Heterozyklus bilden können, der gegebenenfalls weitere Heteroatome enthalten kann.

Diese Polyharnstoff-Systeme zeichnen sich durch eine besonders schnelle Aushärtung aus.

Bei den in Komponente E) verwendeten Verbindungen kann es sich ganz besonders bevorzugt um tertiäre Amin ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}-ethanol, 3,3',3"-(1,3,5-Triazinan-1,3,5-triyl)tris(N,N-dimethyl-propan-1-amin) handeln.

Ganz besonders hohe Aushärtungsgeschwindigkeiten können auch erzielt werden, wenn die Komponente E) 0,2 bis 2,0 Gew.-% Wasser und/oder 0,1 bis 1,0 Gew.-% des tertiäres Amin enthält.

Selbstverständlich können in die Polyharnstoff-Systeme auch pharmakologisch aktive Wirkstoffe wie Analgetika mit und ohne antiinflammatorische Wirkung, Antiphlogistika, antimikrobiell wirksame Substanzen, Antimykotika, antiparasitär wirkende Stoffe eingearbeitet werden.

Das erfindungsgemäße Polyharnstoff-System ist besonders zum Verschließen, Verbinden, Verkleben oder Abdecken von Zellgewebe und insbesondere zur Stillung des Austritts von Blut oder Gewebeflüssigkeiten oder dem Verschluss von Leckagen in Zellgewebe geeignet. Ganz besonders bevorzugt kann es zur Verwendung oder zur Herstellung eines Mittels zum Verschließen, Verbinden, Verkleben oder Abdecken von menschliches oder tierisches Zellgewebe verwendet werden. Mit seiner Hilfe können schnell aushärtende, stark am Gewebe anhaftende, transparente, flexible und biokompatible Klebnähte hergestellt werden.

Noch ein weiterer Gegenstand der Erfindung ist Dosiersystem mit zwei Kammern für ein erfindungsgemäßes Polyharnstoff-System, bei dem in der einen Kammer die Komponente A) und in der anderen Kammer die Komponenten B) und gegebenenfalls die Komponenten C), D) und. E) des Polyharnstoff-Systems enthalten sind. Ein derartiges Dosiersystem eignet sich insbesondere dafür das Polyharnstoff-System als Kleber auf Gewebe zu applizieren.

### Beispiele:

Die Erfindung wird im Folgenden anhand von Beispielen näher erläutert.

### Methoden:

- Molekulargewicht:: Die Molekulargewichte wurden mittels Gelpermeationschromatographie (GPC) wie folgt bestimmt: Die Kalibrierung erfolgt mit Polystyrol-Standards mit Molekulargewichten von Mp 1.000.000 bis 162. Als Eluent wurde Tetrahydrofuran p.A. verwendet. Die folgenden Parameter wurden bei der Doppelmessung eingehalten: Entgasung: Online - Degasser; Durchfluß: 1 ml/Min; Analysenzeit: 45 Minuten; Detektoren: Refraktometer und UV-Detektor; Injektionsvolumen: 100 µl - 200 µl. Die Berechnung der Molmassenmittelwerte M_{w}; Mₙ und Mₚ sowie der Polydispersität M_{w}/Mₙ erfolgte softwaregestützt. Basislinienpunkte und Auswertegrenzen wurden entsprechend der DIN 55672 Teil 1 festgelegt.
- NCO-Gehalt:: Der NCO-Gehalt wurde, wenn nicht ausdrücklich anders erwähnt, volumetrisch gemäß DIN-EN ISO 11909 bestimmt.
- Viskosität:: Die Viskosität wurde nach ISO 3219 bei 23 °C bestimmt.
- Restmonomergehalt:: Der Restmonmergehalt wurde nach DIN ISO 17025 bestimmt.
- NMR:: Die NMR Spektren wurden mit einem Bruker DRX 700 Gerät aufgenommen.

### Substanzen:

### Polyethylenglykol 600 (Aldrich)

### Synthese von Tetraethyl-2,2'-(4-(3-(1,4-diethoxy-1,4-dioxobutan-2-ylamino)propyl)heptan-1,7-diyl)bis(azandiyl)disuccinat (3)

### 4-(2-Cyanoethyl)heptandinitril (1)

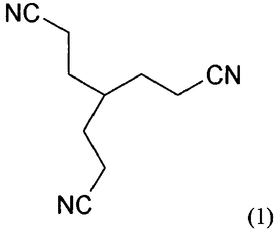

Eine Mischung von 12,2 g (55,4 mmol) 4-(2-cyanoethyl)-4-nitroheptandinitril und 18 ml Tributylzinnhydrid wurde mit 2,78 g Azo-bis-(isobutyronitril) (AIBN) in 250 ml Acetonitril über Nacht zum Rückfluß erhitzt. Nach Entfernen des Lösungsmittels im Vakuum wurde das Produkt mit n-Hexan ausgerührt und mit einer Mischung aus n-Hexan und Dichlormethan kristallisiert. Man erhielt 8,25 g (85%) des Produktes als farblosen Feststoff.

### 4-(3-Aminopropyl)heptan-1,7-diamin (2)

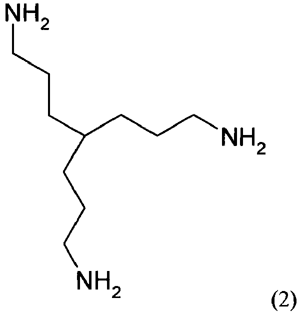

8 g (45,7 mmol) 4-(2-Cyanoethyl)heptandinitril wurden in 100 ml 7N methanolischer AmmoniakLösung 5 h bei 130 °C und 100 bar Wasserstoffdruck hydriert. Nach Abkühlen auf Raumtemperatur wurde die Reaktionsmischung über Kieselgur filtriert und mit Methanol gewaschen. Nach Entfernen des Lösungsmittels im Vakuum wurden 7,5 g des Rohproduktes erhalten und ohne weitere Reinigung in der nächsten Stufe eingesetzt.

### Tetraethyl-2,2'-(4-(3-(1,4-diethoxy-1,4-dioxobutan-2-ylamino)propyl)heptan-1,7-diyl)bis(azandiyl)disuccinat (3)

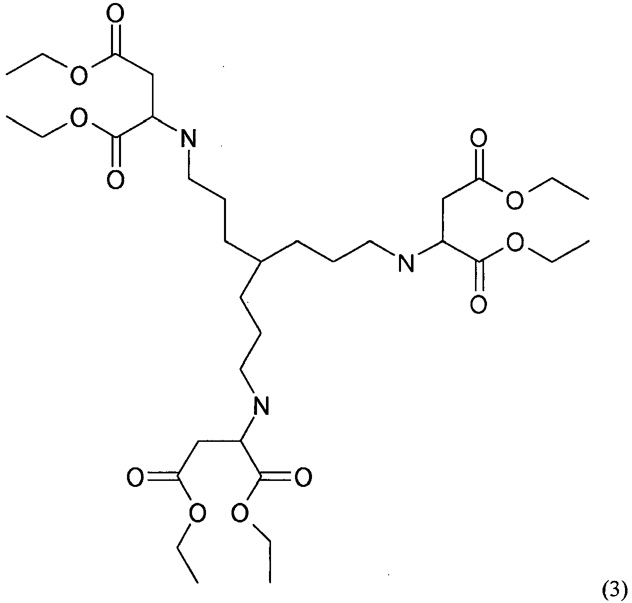

Zu einer Lösung von 7,5 g (40 mmol) 4-(3-Aminopropyl)heptan-1,7-diamin in 100 ml Ethanol wurden 25,7 g (150 mmol) Maleinsäurediethylester gegeben. Die Reaktionsmischung wurde 3 Tage bei 60°C gerührt. Nach Entfernen des Lösemittels im Vakuum wurde das Produkt säulenchromatographisch gereinigt (Hexan/Ethylacetat 1:1, dann Dichlormethan/Methanol 10:1). Es wurden 15,5 g (39 %) des Produktes als gelbes Öl erhalten.

¹H-NMR (CDCl₃, 700 MHz): δ = 1.27 (t, 9H), 1,28 (m, 6H), 1.29 (t, 9H), 1.42 (m, 6H) 1.72 (s, 3NH), 2,48 (m, 1H), 2,61, (m, 6H), 2.64 (dd, 6H), 3.60 (t, 3H), 4.19 (q, 6H), 4.2 (q, 6H).

¹³C-NMR (CDCl₃, 700 MHz): 13.93, 13.99, 26.9, 30.6, 36.9, 37.9, 48.3, 57.6, 60.4, 60.9, 170.6, 173.4.

### Synthese von Prepolymer A

212,5 g (1,8 mol) Bersteinsäure wurden mit 1591,5 g Polyethylenglykol 600 (2,6 mol) unter Rühren auf 235 °C erhitzt. Dabei wurde über 8,5 h das entstehende Wasser abdestilliert. Anschließend wurden 100 ppm Zinn(II)chlorid hinzugegeben und für weitere 9 h unter Vakuum (15 mbar) am Wasserabscheider auf 235 °C erhitzt.

672 g HDI (4 mol) wurden mit 0,1 Gew% Benzoylchlorid vorgelegt und auf 80 °C erhitzt. Anschließend wurde unter Rühren 788 g des Polyesters über 1h zudosiert und bis zum Erreichen eines konstanten NCO-Gehalts bei 80 °C weiter gerührt. Das überschüssige HDI wurde bei 140 °C und 0,13 mbar mittels Dünnschichtverdampfer entfernt. Das erhaltene Prepolymer hatte einen NCO-Gehalt von 3,5 % und eine Viskosität von 4700 mPas/23°C. Der Restmonomerengehalt betrug < 0,03 % **HDI.**

### Synthese von Prepolymer B

Analog zu Prepolymer B wurden aus 263 g (1,8 mol) Adipinsäure und 1591,5 g Polyethylenglykol 600 (2,6 mol) ein Prepolymer hergestellt. Das erhaltene Prepolymer hatte einen NCO-Gehalt von 5,93 % und eine Viskosität von 1450 mPas/23°C. Der Restmonomerengehalt betrug < 0,03 % HDI.

### Synthese von Prepolymer C

465 g HDI und 2.35 g Benzoylchlorid wurden in einem 11 Vierhalskolben vorgelegt. Innerhalb von 2h wurden bei 80 °C 931.8 g eines Polyethers mit einem Ethylenoxidgehalt von 71 % und einem Propylenoxidgehalt von 29 % (jeweils bezogen auf dem gesamten Alkylenoxidgehalt) hinzugefügt und 1 h nachgerührt. Anschließend wurde durch Dünnschichtdestillation bei 130 °C und 0,13 mbar das überschüssige HDI abdestilliert. Man erhielt 980 g (71 %) eines Prepolymers mit einem NCO-Gehalt von 2,37% und einer Viskosität von 4500 mPas/23°C. Der Restmonomerengehalt betrug < 0,03 % HDI.

### Herstellung des Gewebeklebers

4 g des jeweiligen Prepolymers wurden mit einer equivalenten Menge der hergestellten aminofunktionellen Verbindung 3 in einem Becher gut verrührt. Das Polyharnstoff-System wurde unmittelbar danach auf das zu klebende Muskelgewebe als dünne Schicht aufgetragen. Als Verarbeitungszeit wurde dabei die Zeit bestimmt, innerhalb der das Polyharnstoff-System noch eine so niedrige Viskosität besaß, dass es problemlos auf das Gewebe aufgetragen werden könnte.

Die Zeit, nach der das Polyharnstoff-System nicht mehr klebrig war (tack free time) wurde durch Haftversuche mit einem Glasstab gemessen. Dazu wurde der Glasstab mit der Schicht aus dem Polyharnstoff-System in Kontakt gebracht. Blieb dieser nicht mehr haften, wurde die System als tack free angesehen. Zusätzlich wurde die Klebekraft bestimmt, indem zwei Stücke Muskelgewebe (1 = 4 cm, h = 0.3 cm, b = 1 cm) an den Enden 1 cm weit mit dem Polyharnstoff-System bestrichen und überlappend geklebt wurden. Die Klebkraft des Polyharnstoff-Systems wurde jeweils durch Zug überprüft.

| | Härter | Verarbeitungszeit | Tack free time | Klebekraft |
|---|---|---|---|---|
| Prepolymer A | 3 | 1 min 30 s | 2 min | ++ |
| Prepolymer B | 3 | 1 min | 2 min | + |
| Prepolymer B (Vergleichsbeispiel 1) | 4 | 8 min | > 30 min | + |
| Prepolymer A | 5 | 3 min | 5 min | ++ |

| | | | | |
|---|---|---|---|---|
| ++: sehr gut; +: gut | | | | |

### Vergleichsbeispiel 1: Difunktionelles Prepolymer + difunktioneller Härter

Anstelle des hergestellten aminofunktionellen Verbindung 3 wurde das in EP 2 145 634 beschriebene Tetraethyl-2,2'-[(2-methylpentan-1,5-diyl)diimino]dibutandioat (4) mit dem Prepolymer B umgesetzt.. Die Verarbeitungszeit betrug hierbei bei 8 min. Das Polyharnstoff-System war auch nach 10 min noch nicht tack free.

### Messung der Zytotoxizität eines mit (3) hergestellten Klebstoffs

Das Prepolymer C wurde mit einer äquivalenten Menge 3 umgesetzt. Die Zytotoxizität wurde gemäß ISO 10993-5:2009 mit L929 Zellen gemessen. Es hat keine Herabsetzung der Zellviabilität stattgefunden. Das Polyharnstoff-System ist damit nicht als zytotoxisch einzustufen.

### Vergleichsbeispiel 2: Zytotoxizität eines strukturell ähnlichen trifunktionellen Härters

Diethyl-2-[(8-[(1,4-diethoxy-1,4-dioxobutan-2-yl)amino]-4-{[(1,4-diethoxy-1,4-dioxobutan-2-yl)amino]methyl}octyl)amino]butandioat (5)

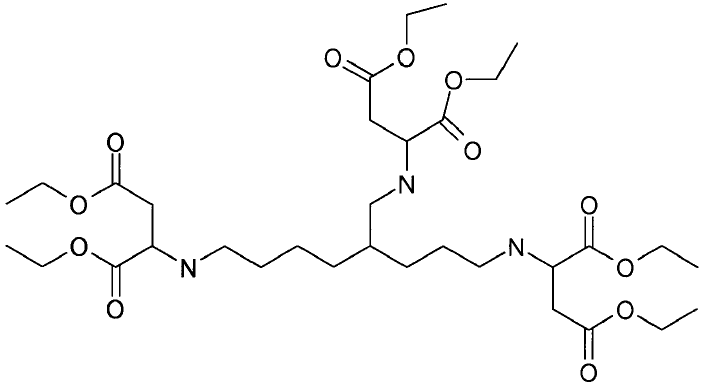

Zu 115,3 g (0,6 Mol) Triaminononan wurden 346 g (2 Mol) Maleinsäurediethylester getropft. Die Reaktionsmischung wurde 3 Tage auf 60°C erwärmt, bis kein Maleinsäureester mehr nachweisbar war (Bayer-Reagenz).

### Messung der Zytotoxizität des ausgehärteten Klebstoffes

4 g des Prepolymers C wurden mit einer equivalenten Menge (5) in einem Becher gut verrührt und ausgehärtet. Der Klebstoff wurde gemäß ISO 10993-5:2009 mit L929 Zellen gemessen. Die Zellviabilität sank auf 4% (hohe Cytotoxizität).

## Patentansprüche

1. Verbindung der Formel (I) 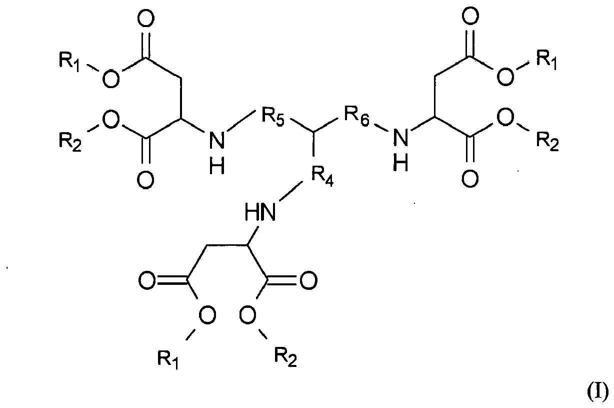 in der
R₁, R₂ jeweils unabhängig voneinander gleiche oder verschiedene organische Reste, die keinen Zerewitinoff-aktiven Wasserstoff aufweisen und
R₄, R₅, R₆ jeweils unabhängig voneinander gesättigte, linearer oder verzweigter, gegebenenfalls auch in der Kette mit Heteroatomen substituierte, organische Reste, die keinen Zerewitinoff-aktiven Wasserstoff aufweisen
sind.

2. Verbindung nach Anspruch 1, **dadurch gekennzeichnet, dass** die Reste R₄, R₅, R₆ jeweils unabhängig voneinander lineare oder verzweigte, insbesondere gesättigte, aliphatische C1 bis C12, bevorzugt C2 bis C10, besonders bevorzugt C3 bis C8 und ganz besonders bevorzugt C3 bis C6 Kohlenwasserstoffreste sind.

3. Verbindung nach einem der Ansprüche 1 oder 2, **dadurch gekennzeichnet, dass** die Reste R₁, R₂ jeweils unabhängig voneinander lineare oder verzweigte organische C1 bis C 10, bevorzugt C1 bis C8, besonders bevorzugt C2 bis C6, ganz besonders bevorzugt C2 bis C4 Reste und insbesondere aliphatische Kohlenwasserstoffreste sind.

4. Verbindung nach einem der Ansprüche 1 bis 3, **dadurch gekennzeichnet, dass** jeweils die Reste R₁ und R₂ gleich und / oder jeweils die Reste R₄, R₅, R₆ gleich sind.

5. Polyharnstoff-System, umfassend
als Komponente A) isocyanatfunktionelle Prepolymere erhältlich durch Umsetzung von
aliphatischen Polyisocyanaten A1) mit
Polyolen A2), die insbesondere ein zahlenmittleres Molekulargewicht von ≥ 400 g/mol und eine mittlere OH-Funktionalität von 2 bis 6 aufweisen können,
als Komponente B) aminofunktionelle Verbindung gemäß einem der Ansprüche 1 bis 4,
gegebenenfalls als Komponente C) organische Füllstoffe, die insbesondere eine nach DIN 53019 gemessenen Viskosität bei 23 °C im Bereich von 10 bis 6000 mPas aufweisen können,
gegebenenfalls als Komponente D) Umsetzungsprodukte von isocyanatfunktionellen Prepolymeren gemäß Komponente A) mit aminofunktionellen Verbindungen gemäß Komponente B) und/oder organischen Füllstoffen gemäß Komponente C) und
gegebenenfalls als Komponente E) Wasser und/oder ein tertiäres Amin.

6. Polyharnstoff-System nach Anspruch 5, **dadurch gekennzeichnet, dass** die Polyole A2) Polyesterpolyole und/oder Polyester-Polyether-Polyole und/oder Polyetherpolyole, insbesondere Polyester-Polyether-Polyole und/oder Polyetherpolyole mit einem Ethylenoxidanteil zwischen 60 und 90 Gew.-%, enthalten.

7. Polyharnstoff-System nach einem der Ansprüche 5 oder 6, **dadurch gekennzeichnet, dass** die Polyole A2) ein zahlenmittleres Molekulargewicht von 4000 bis 8500 g/mol aufweisen.

8. Polyharnstoff-System nach einem der Ansprüche 5 bis 7, **dadurch gekennzeichnet, dass** die Prepolymere A) eine mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 aufweisen.

9. Polyharnstoff-System nach einem der Ansprüche 5 bis 8, **dadurch gekennzeichnet, dass** es sich bei den organischen Füllstoffen der Komponente C) um hydroxyfunktionelle Verbindungen, insbesondere um Polyetherpolyole mit sich wiederholenden Ethylenoxid Einheiten handelt.

10. Polyharnstoff-System nach Anspruch 9, **dadurch gekennzeichnet, dass** die Füllstoffe der Komponente C) eine mittlere OH-Funktionalität von 1,5 bis 3, bevorzugt von 1,8 bis 2,2 und besonders bevorzugt von 2 aufweisen.

11. Polyharnstoff-System nach einem der Ansprüche 5 bis 10, **dadurch gekennzeichnet, dass** die Komponente E) ein tertiäre Amine der allgemeinen Formel (II) 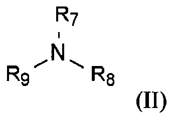 enthält, in der
R₇, R₈, R₉ unabhängig voneinander Alkyl- oder Heteroalkyreste mit Heteroatomen in der Alkylkette oder an deren Ende sein können, oder R₇ und R₈ gemeinsam mit dem sie tragenden Stickstoffatom einen aliphatischen, ungesättigten oder aromatischen Heterozyklus bilden können, der gegebenenfalls weitere Heteroatome enthalten kann.

12. Polyharnstoff-System nach einem der Ansprüche 5 bis 11, **dadurch gekennzeichnet, dass** das tertiäre Amin ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol, 3,3',3"-(1,3,5-Triazinan-1,3,5-triyl)tris(N,N-dimethyl-propan-1-amin) ist.

13. Polyharnstoff-System nach einem der Ansprüche 5 bis 12, **dadurch gekennzeichnet, dass** Komponente E) 0,2 bis 2,0 Gew.-% Wasser und/oder 0,1 bis 1,0 Gew.-% des tertiäres Amin enthält.

14. Polyharnstoff-System nach einem der Ansprüche 5 bis 13 zum Verschließen, Verbinden, Verkleben oder Abdecken von Zellgewebe, insbesondere zur Stillung des Austritts von Blut oder Gewebeflüssigkeiten oder dem Verschluss von Leckagen in Zellgewebe.

15. Polyharnstoff-System nach Anspruch 14 zur Verwendung zum Verschließen, Verbinden, Verkleben oder Abdecken von menschliches oder tierisches Zellgewebe.

16. Dosiersystem mit zwei Kammern für ein Polyharnstoff-System nach einem der Ansprüche 5 bis 15, **dadurch gekennzeichnet, dass** in der einen Kammer die Komponente A) und in der anderen Kammer die Komponenten B) und gegebenenfalls die Komponenten C), D) und. E) des Polyharnstoff-Systems enthalten sind.

## Claims

1. Compound of formula (I) 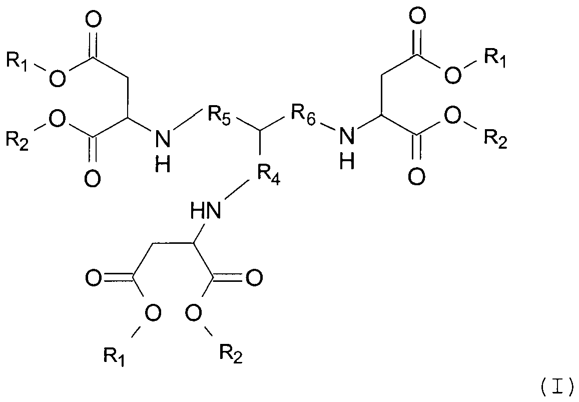 wherein
R₁, R₂ each independently of the other are identical or different organic radicals which do not contain Zerewitinoff-active hydrogen, and
R₄, R₅, R₆ each independently of the others are saturated, linear or branched organic radicals which do not contain Zerewitinoff-active hydrogen and which are also optionally substituted in the chain by heteroatoms.

2. Compound according to claim 1, **characterised in that** the radicals R₄, R₅, R₆ each independently of the others are linear or branched, in particular saturated, aliphatic C1 to C12, preferably C2 to C10, particularly preferably C3 to C8 and most particularly preferably C3 to C6 hydrocarbon radicals.

3. Compound according to either claim 1 or claim 2, **characterised in that** the radicals R₁, R₂ each independently of the other are linear or branched organic C1 to C10, preferably C1 to C8, particularly preferably C2 to C6, most particularly preferably C2 to C4 radicals and in particular are aliphatic hydrocarbon radicals.

4. Compound according to any one of claims 1 to 3, **characterised in that** the radicals R₁ and R₂ are each identical and/or the radicals R₄, R₅, R₆ are each identical.

5. Polyurea system comprising
as component A) isocyanate-functional prepolymers obtainable by reaction of
aliphatic polyisocyanates A1) with
polyols A2), which in particular can have a number-average molecular weight of ≥ 400 g/mol and a mean OH functionality of from 2 to 6,
as component B) an amino-functional compound according to any one of claims 1 to 4,
optionally as component C) organic fillers, which in particular can have a viscosity at 23°C, measured in accordance with DIN 53019, in the range of from 10 to 6000 mPas,
optionally as component D) reaction products of isocyanate-functional prepolymers according to component A) with amino-functional compounds according to component B) and/or organic fillers according to component C), and
optionally as component E) water and/or a tertiary amine.

6. Polyurea system according to claim 5, **characterised in that** the polyols A2) comprise polyester polyols and/or polyester-polyether polyols and/or polyether polyols, in particular polyester-polyether polyols and/or polyether polyols having an ethylene oxide content of from 60 to 90 wt.%.

7. Polyurea system according to either claim 5 or claim 6, **characterised in that** the polyols A2) have a number-average molecular weight of from 4000 to 8500 g/mol.

8. Polyurea system according to any one of claims 5 to 7, **characterised in that** the prepolymers A) have a mean NCO functionality of from 1.5 to 2.5, preferably of from 1.6 to 2.4, more preferably of from 1.7 to 2.3, most particularly preferably of from 1.8 to 2.2 and in particular of 2.

9. Polyurea system according to any one of claims 5 to 8, **characterised in that** the organic fillers of component C) are hydroxy-functional compounds, in particular polyether polyols having repeating ethylene oxide units.

10. Polyurea system according to claim 9, **characterised in that** the fillers of component C) have a mean OH functionality of from 1.5 to 3, preferably of from 1.8 to 2.2 and particularly preferably of 2.

11. Polyurea system according to any one of claims 5 to 10, **characterised in that** component E) comprises a tertiary amine of the general formula (II) 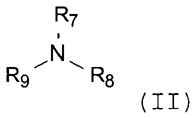 wherein
R₇, R₉ independently of one another can be alkyl or heteroalkyl radicals having heteroatoms in the alkyl chain or at the end thereof, or R₇ and R₈ together with the nitrogen atom carrying them can form an aliphatic, unsaturated or aromatic heterocycle which can optionally contain further heteroatoms.

12. Polyurea system according to any one of claims 5 to 11, **characterised in that** the tertiary amine is selected from the group triethanolamine, tetrakis(2-hydroxyethyl)ethylenediamine, N,N-dimethyl-2-(4-methylpiperazin-1-yl)ethanamine, 2-{[2-(dimethylamino)ethyl](methyl)amino}ethanol, 3,3',3"-(1,3,5-triazinane-1,3,5-triyl)tris(N,N-dimethyl-propan-1-amine).

13. Polyurea system according to any one of claims 5 to 12, **characterised in that** component E) comprises from 0.2 to 2.0 wt.% water and/or from 0.1 to 1.0 wt.% of the tertiary amine.

14. Polyurea system according to any one of claims 5 to 13 for sealing, bonding, gluing or covering cell tissue, in particular for stopping the escape of blood or tissue fluids or for sealing leaks in cell tissue.

15. Polyurea system according to claim 14 for use for sealing, bonding, gluing or covering human or animal cell tissue.

16. Metering system having two chambers for a polyurea system according to any one of claims 5 to 15, **characterised in that** one chamber contains component A) and the other chamber contains component B) and optionally components C), D) and E) of the polyurea system.

## Revendications

1. Composé de formule (I) 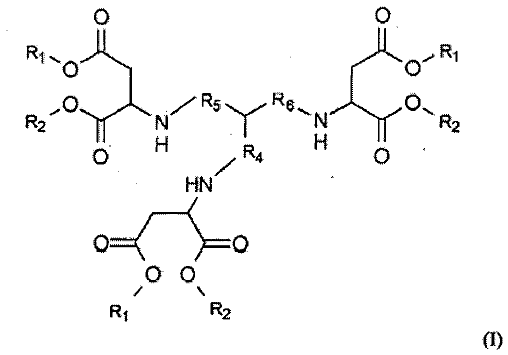 dans laquelle
R₁, R₂ représentent chacun, indépendamment l'un de l'autre, des radicaux organiques identiques ou différents, qui ne comportent pas d'hydrogène actif selon la méthode de Zerewitinoff et
R₄, R₅, R₆ représentent chacun indépendamment des radicaux organiques saturés, linéaires ou ramifiés, éventuellement également substitués dans la chaîne par des hétéroatomes, qui ne comportent pas d'hydrogène actif selon la méthode de Zerewitinoff.

2. Composé selon la revendication 1, **caractérisé en ce que** les radicaux R₄, R₅, R₆ représentent chacun indépendamment des radicaux hydrocarbonés linéaires ou ramifiés, en particulier saturés, aliphatiques en C₁-C₁₂, de préférence en C₂-C₁₀, de façon particulièrement préférée en C₃-C₈ et de façon tout particulièrement préférée en C₃-C₆.

3. Composé selon l'une quelconque des revendications 1 et 2, **caractérisé en ce que** les radicaux R₁, R₂ représentent chacun, indépendamment l'un de l'autre, des radicaux organiques linéaires ou ramifiés en C₁-C₁₀, de préférence en C₁-C₈, de façon particulièrement préférée en C₂-C₆, de façon tout particulièrement préférée en C₂-C₄ et en particulier des radicaux hydrocarbonés aliphatiques.

4. Composé selon l'une quelconque des revendications 1 à 3, **caractérisé en ce que**, respectivement, les radicaux R₁ et R₂ sont identiques et/ou, respectivement, les radicaux R₄, R₅, R₆ sont identiques.

5. Système polyurée, comprenant
en tant que composant A) des prépolymères à fonction isocyanate, pouvant être obtenus par mise en réaction de
polyisocyanates aliphatiques A1) avec
des polyols A2) qui peuvent présenter en particulier une masse moléculaire moyenne en nombre de ≥ 400 g/mole et une fonctionnalité OH moyenne de 2 à 6,
en tant que composant B) un composé à fonction amino selon l'une quelconque des revendications 1 à 4,
éventuellement en tant que composant C) des charges organiques qui peuvent en particulier présenter une viscosité à 23 °C, mesurée selon DIN 53019, dans la plage de 10 à 6 000 mPa.s,
éventuellement en tant que composant D) des produits de réaction de prépolymères à fonction isocyanate selon le composant A) avec des composés à fonction amino selon le composant B) et/ou des charges organiques selon le composant C) et
éventuellement en tant que composant E) de l'eau et/ou une amine tertiaire.

6. Système polyurée selon la revendication 5, **caractérisé en ce que** les polyols A2) comprennent des polyesterpolyols et/ou des polyester-polyéther-polyols et/ou des polyétherpolyols, en particulier des polyester-polyéther-polyols et/ou des polyétherpolyols ayant une teneur en oxyde d'éthylène comprise entre 60 et 90 % en poids.

7. Système polyurée selon l'une quelconque des revendications 5 et 6, **caractérisé en ce que** les polyols A2) ont une masse moléculaire moyenne en nombre de 4 000 à 8 500 g/mole.

8. Système polyurée selon l'une quelconque des revendications 5 à 7, **caractérisé en ce que** les prépolymères A) présentent une fonctionnalité NCO moyenne de 1,5 à 2,5, de préférence de 1,6 à 2,4, de façon particulièrement préférée de 1,7 à 2,3, de façon tout particulièrement préférée de 1,8 à 2,2 et en particulier de 2.

9. Système polyurée selon l'une quelconque des revendications 5 à 8, **caractérisé en ce que** pour ce qui est des charges organiques du composant C) il s'agit de composés à fonction hydroxy, en particulier de polyétherpolyols à motifs oxyde d'éthylène répétitifs.

10. Système polyurée selon la revendication 9, **caractérisé en ce que** les charges du composant C) présentent une fonctionnalité OH moyenne de 1,5 à 3, de préférence de 1,8 à 2,2 et de façon particulièrement préférée de 2.

11. Système polyurée selon l'une quelconque des revendications 5 à 10, **caractérisé en ce que** le composant E) contient une amine tertiaire de formule générale (II) 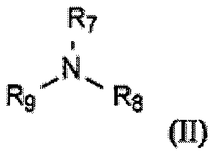 dans laquelle
R₇, R₈, R₉ peuvent être chacun indépendamment des radicaux alkyle ou hétéroalkyle comportant des hétéroatomes dans la chaîne alkyle ou à son extrémité, ou R₇ et R₈ peuvent former ensemble avec l'atome d'azote qui les porte un hétérocycle aliphatique, insaturé ou aromatique, qui peut éventuellement contenir d'autres hétéroatomes.

12. Système polyurée selon l'une quelconque des revendications 5 à 11, **caractérisé en ce que** l'amine tertiaire est choisie dans le groupe constitué par la triéthanolamine, la tétrakis(2-hydroxyéthyl)éthylène-diamine, la N,N-diméthyl-2-(4-méthylpipérazin-1-yl)-éthanamine, le 2-{[2-(diméthylamino)éthyl](méthyl)-amino)éthanol, la 3,3',3"-(1,3,5-triazinane-1,3,5-triyl)tris(N,N-diméthyl-propan-1-amine).

13. Système polyurée selon l'une quelconque des revendications 5 à 12, **caractérisé en ce que** le composant E) contient 0,2 à 2,0 % en poids d'eau et/ou 0,1 à 1,0 % en poids de l'amine tertiaire.

14. Système polyurée selon l'une quelconque des revendications 5 à 13, destiné à la fermeture, au jointoiement, au collage ou au recouvrement de tissu cellulaire, en particulier pour arrêter l'épanchement de sang ou de liquides tissulaires ou pour la fermeture de brèches dans un tissu cellulaire.

15. Système polyurée selon la revendication 14, destiné à l'utilisation pour la fermeture, le jointoiement, le collage ou le recouvrement de tissu cellulaire humain ou animal.

16. Système doseur comportant deux compartiments pour un système polyurée selon l'une quelconque des revendications 5 à 15, **caractérisé en ce que** dans un compartiment est contenu le composant A) et dans l'autre compartiment est contenu le composant B) et sont éventuellement contenus les composants C), D) et E) du système polyurée.
